(19) Europäisches Patentamt
European Patent Office
Office européen des brevets

(11) Publication number: **0 178 152 B1**

(12) **EUROPEAN PATENT SPECIFICATION**

(45) Date of publication of patent specification: **25.09.91**

(51) Int. Cl.5: **C12P 21/04**, A61K 37/02, C12N 1/20, //(C12P21/04, C12R1:465)

(21) Application number: **85307185.0**

(22) Date of filing: **08.10.85**

(54) **Production of A-21978C derivatives.**

(30) Priority: **09.10.84 US 658979**

(43) Date of publication of application:
**16.04.86 Bulletin 86/16**

(45) Publication of the grant of the patent:
**25.09.91 Bulletin 91/39**

(84) Designated Contracting States:
**AT BE CH DE FR GB IT LI LU NL SE**

(56) References cited:
**EP-A- 0 010 421**
**EP-A- 0 095 295**

(73) Proprietor: **ELI LILLY AND COMPANY**
**Lilly Corporate Center**
**Indianapolis Indiana 46285(US)**

(72) Inventor: **Huber, Floyd Milton**
**Rural Route No. 6 Box 212**
**Danville Indiana 46122(US)**
Inventor: **Pieper, Richard Louis**
**8836 Rocky Ridge Road**
**Indianapolis Indiana 46217(US)**
Inventor: **Tietz, Anthony Joseph**
**5003 Vantage Point Road**
**Plainfield Indiana 46168(US)**
Inventor: **Eaton, Tom Edward**
**3991 Santa Clara Drive**
**Greenwood Indiana 46142(US)**
Inventor: **Ford, Lynda Maxine**
**3939 North Lesley Avenue**
**Indianapolis Indiana 46226(US)**
Inventor: **Godfrey, Otis Webster, Jr.**
**222 Serenity Way**
**Greenwood Indiana 46142(US)**
Inventor: **Huber, Mary Louise Braun**
**Rural Route No. 6 Box 212**
**Danville Indiana 46122(US)**
Inventor: **Zmijewski, Milton Joseph, Jr.**
**10152 Partridge Place**
**Carmel Indiana 46032(US)**

(74) Representative: **Hudson, Christopher Mark et al**
**Erl Wood Manor**
**Windlesham Surrey GU20 6PH(GB)**

**EP 0 178 152 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to an improved process for preparing derivatives of the A-21978C cyclic peptide antibiotics of formula (I):

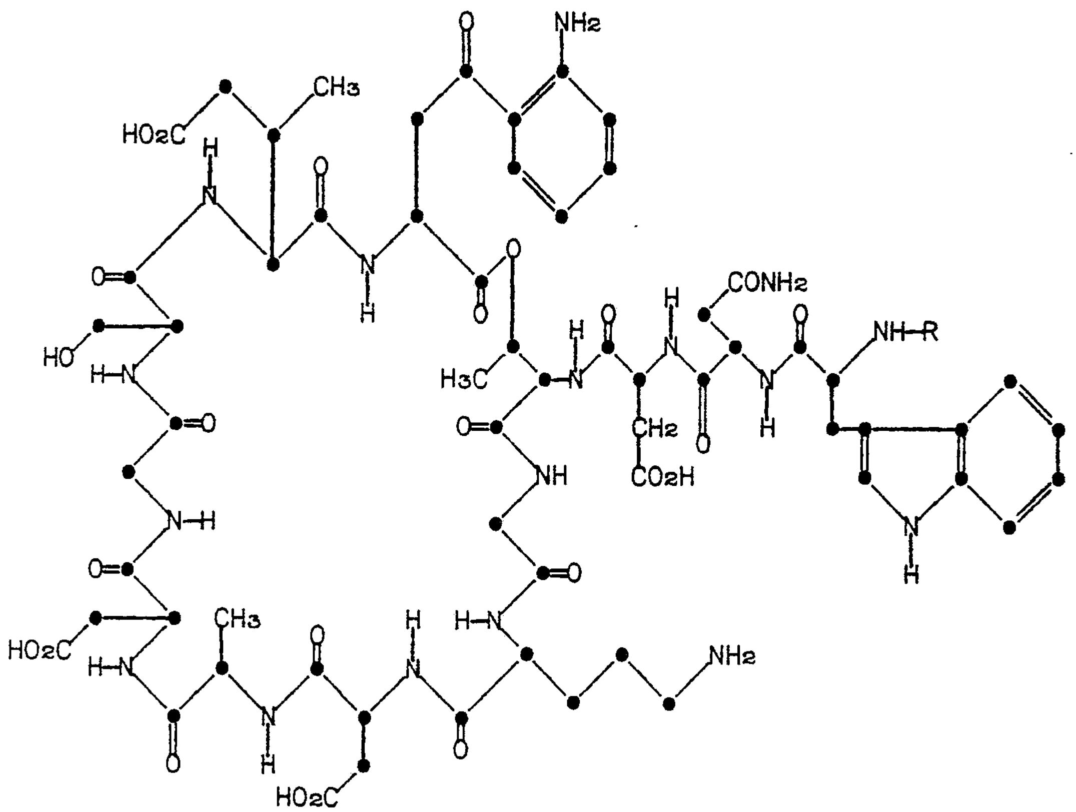

I

in which R is $C_2$-$C_{14}$-alkanoyl. In particular, the improved process comprises feeding a $C_2$-$C_{14}$-alkanoic acid to the A-21978C producing culture during the fermentation. The advantages of this process are: 1) fewer steps are involved than previous processes, 2) the product yield is increased; and 3) less time is required.

The A-21978C family of antibiotics are excellent antibacterial agents. A particularly important group of A-21978C derivatives are those of formula I (see also, Bernard J. Abbott, David S. Fukuda and Manuel Debono, U.S. Patent No. 4,537,717). Previously, preparation of these derivatives required a multistep process, which was time-consuming, yield-consuming and expensive. The present invention provides an improved process for making these A-21978C derivatives directly. The prior process for preparing a formula I derivative, such as the n-decanoyl derivative of A-21978C, required the following steps:

1. Fermentation of the A-21978C-producing culture.
    a. Initiating with a liquid nitrogen ampoule.
    b. Primary inoculum stage (48 hours).
    c. Secondary inoculum stage (24 hours).
    d. Tertiary inoculum stage (24 hours).
    e. Fermentation (140 hours).
2. Filtration, resin adsorption and elution, and concentration.
3. Preparation of t-Boc (t-butoxycarbonyl protected) complex.
4. Concentration of the protected complex.
5. Fermentation of the deacylating culture, using e.g. Actinoplanes utahensis.
    a. Initiating with a liquid nitrogen ampoule
    b. Primary inoculum stage (72 hours)
    c. Secondary inoculum stage (48 hours)

d. Fermentation (67 hours)

6. Deacylation of the complex with the deacylating culture.
7. Filtration, resin adsorption and elution, and concentration.
8. Reacylation.
9. Hydrolysis of the protecting group.
10. Final purification.

The present novel process comprises adding a $C_2$-$C_{14}$ alkanoic acid (an ROH compound in which R is as defined supra), or an ester or salt thereof, to an A-21978C-producing culture during the production stage of the fermentation (step 1e) to give the corresponding formula I compound. With this process, steps 3, 4, 5, 6, 7, 8 and 9 of the previous process can be eliminated. In addition, the new process substantially increases the yields obtained over those obtained using the previous process.

Streptomyces roseosporus strains NRRL 11379 (A-21978.6) and NRRL 15998 (A-21978.65), a mutant strain of NRRL 11379, are useful A-21978C-producing cultures. These cultures are part of the stock culture collection of the Northern Regional Research Center, U.S. Department of Agriculture, Agricultural Research Service, Peoria, Illinois 61604, from which they are available to the public under the accession numbers NRRL 11379 and NRRL 15998. The S. roseosporus NRRL 11379 culture and conditions for its use in the production of the A-21978C antibiotics are described by Robert L. Hamill and Marvin M. Hoehn in U.S. Patent 4,331,594, incorporated herein by reference.

Although the microorganism strains preferred in the present process are those designated A-21978.6 or A-21978.65, any A-21978C-producing culture may be used. Those skilled in the art will recognize which strains will be useful for this purpose.

The naturally occurring A-21978C factors described in U.S. 4,331,594 are factors $C_0$,$C_1$,$C_2$,$C_3$,$C_4$ and $C_5$. In factors $C_1$,$C_2$,$C_3$,$C_4$ and $C_5$, the R in formula I is a specific $C_{10}$-$C_{12}$-alkanoyl group. A-21978C factor $C_0$, earlier thought to have a unique branched $C_{10}$-alkanoyl side chain, has been found to be a mixture of two components in approximately a 2:1 ratio. The major component has a branched-$C_{10}$-side chain, and the minor component has the straight-$C_{10}$-side chain.

For convenience in discussion, when a formula I compound is prepared by the process of this invention, it is also called an A-21978C factor. Except for the naturally-occurring factors, the length of the side chain is used to designate the factor. Thus, for example, the formula I compound in which R = octanoyl, when prepared by this process, is called an A-21978$C_8$ factor.

The alkyl portion of the $C_2$-$C_{14}$ alkanoic acid, ester or salt (the substrate) used in the process of this invention can be a straight or branched chain. To prepare the naturally occurring A-21978C factors $C_1$, $C_2$ or $C_3$, for example, an 8-methyldecanoic, 10-methyl-dodecanoic or 10-methylundecanoic acid, ester or salt would be used. The $C_2$-$C_{14}$ straight-chain acids, esters and salts are recommended for use in the process because of their availability and lower cost. An especially preferred substrate is n-decanoic acid, its esters or salts.

When using a $C_2$-$C_{14}$ alkanoic acid ester, the $C_1$-$C_4$-alkyl esters are preferred. In such an ester, the $C_1$-$C_4$-alkyl group also may be straight or branched chain.

Representative suitable salts of $C_2$-$C_{14}$-alkanoic acids which may be used in the process include those formed from alkali metals and alkaline-earth metals such as sodium, potassium, lithium, cesium, rubidium, barium, calcium or magnesium. Suitable amine salts include the ammonium, the primary, secondary and tertiary $C_1$-$C_4$-alkyl- ammonium and hydroxy-$C_2$-$C_4$-alkyl-ammonium salts.

Preferably, the substrate is added to the fermentation in the form of a sterile solution. A particularly useful solvent for this purpose is methyl oleate, although other solvents such as ethanol, ethyl acetate and $C_1$-$C_4$ esters of unsaturated fatty acids can be used. If the substrate is suitably fluid at the fermentation temperature, it may be added directly.

The rate of addition of the substrate must be low enough to avoid producing a toxic effect on the fermentation, but high enough to increase the yield of the desired formula I compound. Rates of addition of about 0.05 to about 0.5 ml per liter of fermentation broth per hour are recommended. A rate of from about 0.1 to about 0.2 ml per liter of fermentation broth per hour is preferred.

The substrate is added to the growing A-21978C-producing culture during the production stage of the fermentation, beginning at from about 15 to about 32 hours and continuing until the fermentation is terminated. The substrate can be added by various methods, but preferably it is added by a method which best approaches a steady flow.

Following the fermentation, if desired, the formula I compound produced can be recovered using procedures known in the art (see, e.g., U.S. Patent 4,331,594).

This invention also provides the previously mentioned new microorganism, Streptomyces roseosporus NRRL 15998. This microorganism also produces the A-21978C antibiotics. In particular, this invention

relates to an improved process for producing the A-21978C antibiotics by culturing the novel strain of Streptomyces roseosporus, NRRL 15998, under submerged aerobic fermentation conditions, until a substantial level of the A-21978C antibiotics is produced. The A-21978C antibiotic complex may be extracted from the fermentation broth and from the mycelium with polar organic solvents. The A-21978C individual factors also may be separated and further purified by techniques known in the art such as column chromatography.

Commonly, the culture isolated from the natural state (the "wild type") produces the antibiotic in low yield. Often, antibiotic production is erratic. Strains with enhanced potency and strains which consistently produce the antibiotic are, therefore, of great value.

The present aspect of the invention provides a greatly improved process for preparing the A-21978C antibiotics by cultivating Streptomyces roseosporus NRRL 15998, or an A-21978C-producing mutant, variant or recombinant thereof, in a culture medium containing assimilable sources of carbon, nitrogen and inorganic salts, under submerged aerobic conditions, until the A-21978C antibiotics are produced. The A-21978C antibiotic complex, or individual factors may be recovered using various isolation and purification procedures understood in the art.

The microorganism of this portion of the invention has been designated A21978.65. This strain was developed by strain selection and mutation from a culture designated A-21978.6 (NRRL 11379). The A-21978.6 strain, which was studied and characterized by Frederick P. Mertz and Ralph E. Kastner of the Lilly Research Laboratories, was in turn developed from a parent strain isolated from soil from Mount Ararat in Turkey. The A-21978.6 strain was classified as a novel strain of Streptomyces roseosporus, Faicão de Morias and Daliá Maia 1961. This classification was made after comparison with published descriptions [R.E. Buchanan and N.E. Gibbons, "Bergey's Manual of Determinative Bacteriology," The Williams and Wilkins Company, 8th Ed., 1974; and E.B. Shirling and D. Gottlieb, "Cooperative Description of Type Strains of Streptomyces," Intern. Journal of Systematic Bacteriol., 808-809 (1972)].

The classification was based on methods recommended for the International Streptomyces Project [E.B. Shirling and D. Gottlieb, "Methods of Characterization of Streptomyces Species," Intern. Journal of Systematic Bacteriol. 16, 313-340 (1966)] along with certain supplementary tests. Carbon utilization was determined on ISP #9 basal medium to which carbon sources were added to equal a final concentration of 1.0%. The carbon sources were sterilized by filtration; the basal medium was sterilized by autoclaving. Plates were read after 14 days incubation at 30°C. The cell-wall sugars were determined using a modification of the procedure of Lechevalier, (M. P. Lechevalier, "Chemical Methods as Criteria for the Separation of Actinomycetes into Genera," Workshop sponsored by the Subcommittee on Actinomycetes of the American Society cf Microbiology, Dr. Thomas G. Pridham, Convenor; held at the Institute of Microbiology, Rutgers University, The State University of New Jersey, New Brunswick, New Jersey, 1971.) The isomer of diaminopimelic acid was determined using the method of Becker et al. [B. Becker, et al., "Rapid Differentiation Between Norcardia and Streptomyces by Paper Chromatography of Whole Cell Hydrolysates," Appl. Microbiol. 11, 421-423 (1964)]. Amino acid analysis was determined with washed cell-wall fragments. Melanoid pigments were determined using ISP #1 (tryptone-yeast extract broth), ISP #6 (peptone-yeast extract iron agar), ISP #7 (tyrosine agar), ISP #7 modified (ISP #7 without tyrosine), and a tyrosine assay [Yuzuru Mikami, et al., "Modified Arai and Mikani Melanin Formation Test of Streptomyces," Intern. Journal of Systematic Bacteriol. 27(3), 290 (1977)]. Starch hydrolysis was determined by testing for the presence of starch with iodine.

Temperature range, NaCl tolerance, pH range, and antibiotic sensitivity were done using ISP #2 agar medium. The range of temperatures were: 25, 28, 30, 34, 37, 40, 45, 50 and 55°C. NaCl tolerance was measured by adding NaCl to the agar to egual: 0, 1, 2, 3, 4, 5, 6, 8, 10 and 12%. These were incubated at 30°C. The pH range was measured by adjusting the agar from pH 3.0 to 11.0 at increments of 1.0 pH units, just prior to pouring. Antibiotic sensitivity was determined using sensitivity discs padded onto seeded agar plates.

Color names were assigned according to the ISCC-NBS method (K.L. Kelly and D.B. Judd, "The ISCC-NBS Methods of Designating Colors and a Dictionary of Color Names," U.S. Department of Commerce Circ. 553, Washington, D.C., 1955).

Figures in parentheses refer to the Tresner and Backus color series [H.D. Tresner, and E.J. Backus, "System of Color Wheels for Streptomycete Taxonomy," Appl. Microbiol. 11, 335-338 (1956)]. Color tab designations are underlined. The Maerz and Paul color blocks are enclosed in brackets (A. Maerz and M.R. Paul, "Dictionary of Color," McGraw-Hill Book Company, Inc., New York, N.Y., 1950).

Morphology

The morphology of culture A-21978.6 consists of sporophores which are of the Rectus-Flexibilis (RF) classification. Spore chains have >10 spores per chain. Spore surface is smooth.

Culture A-21978.6 is characterized by the production of a predominantly red aerial spore mass color, with a reddish-brown reverse color. A light-brown water-soluble pigment is also present. These characteristics are exhibited on three of 14 agar plating media (ISP #2, ISP #7, TPO). These three media are the only ones which supported abundant aerial and vegetative growth.

Two agar plating media, ISP #4 and glucose-asparagine agar, produced a white-to-gray aerial spore mass color, with a yellow reverse color. No water-soluble pigment was observed. These two media supported good, but not abundant, aerial and vegetative growth.

Nine other agar plating media were used, but these gave poor-to-no growth and sporulation. Aerial color when present, although poor, was in the white-to-gray color series.

Melanoid pigments are absent. Major constituents of the cell wall are: LL-DAP, glycine, glucose, and ribose. This indicates a Type I cell wall, and type C sugar pattern (R. E. Buchanan, and N. E. Gibbons, Eds., "Bergey's Manual of Determinative Bacteriology," The Williams & Wilkins Company, 8th Edition, 1974, p. 658).

The following five cultures were compared in laboratory tests to A-21978.6:

Streptomyces albovinaceous ISP 5136; ATCC 15833
Streptomyces candidus ISP 5141; ATCC 19891
Streptomyces moderatus ISP 5529; ATCC 23443
Streptomyces roseosporus ISP 5122; ATCC 23958
Streptomyces setonii ISP 5395; ATCC 25497

These cultures belong to the white and red color series, have RF type sporophore morphology, smooth spore surface ornamentation, and, according to the ISP descriptions, are melanin negative and do not have a distinctive reverse color or water-soluble pigments. These characteristics, together with carbon-utilization pattern and other secondary features, match those of culture A-21978.6.

When these cultures were compared with A-21978.6 under laboratory conditions, four were rejected. S. candidus and S. setonii exhibited a yellow aerial spore mass on many media, thereby differing from culture A-21978.6. S. albovinaceous and S. moderatus exhibited dark distinctive reverse color, water-soluble pigments, and produced melanoid pigments, all of which were different from culture A-21978.6. The ISP description of S. moderatus refers to reddish brown or strong-brown reverse color, but does not refer to such a characteristic for S. albovinaceous. Neither culture is listed as melanin positive.

Culture A21978.6 was classified, therefore, as a strain of Streptomyces roseosporus, Falcão de Morias and Daliá Maia 1961. This classification was based on comparison with published descriptions and direct laboratory comparisons. The following cultural characteristics summarize the direct comparison studies.

## Cultural Characteristics

### Morphology

| A21978.6 | S. roseosporus |
|---|---|

Sporophores straight to flexuous (RF), with no hooks, loops or spirals observed. Chains of spores >10. The spore surface smooth as determined by scanning electron microscopy.

| | A21978.6 | S. roseosporus |
|---|---|---|
| Spores: | Oblong to oval | Oblong to cylindrical |
| Average: | 0.85 x 1.78 μM | 1.01 x 2.47 μM |
| Range: | 0.65-0.97 x 0.97-2.6 μM | 0.97-1.3 x 1.63 - 3.25 μM |

## Cultural Characteristics (cont)

| | A21978.6 Growth | A21978.6 Color | S. roseosporus Growth | S. roseosporus Color |
|---|---|---|---|---|
| **Carrot Plugs** | | | | |
| Aerial | good | gray c̄ pink | none | none |
| Vegetative | abundant | brown<br>no soluble pigment | good | yellow-brown<br>no soluble pigment |
| **Potato Plugs** | | | | |
| Aerial | good | gray c̄ pink | none | none |
| Vegetative | abundant | brown<br>dark brown soluble pigment | fair | orange-brown<br>no soluble pigment |
| **ISP #1 (Tryptone-yeast ext. agar)** | | | | |
| Aerial | fair | (W)a white | poor | (W)a white |
| Vegetative | good | [10A1] pale yellow green<br>no soluble pigment | poor | [10B2] pale yellow green<br>no soluble pigment |
| **ISP #2 (Yeast-malt extract agar)** | | | | |
| Aerial | abundant | (R) 5cb gy. yellow pink | abundant | (R) 3ca pale orange yellow |
| Vegetative | abundant | [5D10] lt. red brown<br>light brown soluble pigment | abundant | [12L7] lt. olive brown<br>light brown soluble pigment |

Cultural Characteristics (cont)

| | A21978.6 Growth | A21978.6 Color | S. roseosporus Growth | S. roseosporus Color |
|---|---|---|---|---|
| **ISP #3 (Oatmeal agar)** | | | | |
| Aerial | fair | (W)a white | poor | (W)a white |
| Vegetative | fair | [10A2] pale yellow pink light brown soluble pigment | fair | pale greenish gray no soluble pigment |
| **ISP #4 (Inorganic salts starch agar)** | | | | |
| Aerial | good | (W)b white | good | (R) 3c2 pale orange yellow |
| Vegetative | good | [10B1] pale yellow-green light brown soluble pigment | abundant | [11I5] grayish yellow no soluble pigment |
| **ISP #5 (Glycerol - asparagine agar)** | | | | |
| Aerial | fair | (W) 13ba purplish white | fair | (W) b white |
| Vegetative | good | [3B7] gy. yellow pink gy. pink soluble pigment | good | [10C2] grayish yellow light brown soluble pigment |
| **ISP #7 (Tyrosine agar)** | | | | |
| Aerial | abundant | (R) 5cb gy. yellow pink | abundant | (R) 5cb gy. yellow pink |
| Vegetative | abundant | [7L12] mod. red brown dark brown soluble pigment | abundant | [11E5] yellow-brown light brown soluble pigment |

## Cultural Characteristics (cont)

| | A21978.6 Growth | A21978.6 Color | S. roseosporus Growth | S. roseosporus Color |
|---|---|---|---|---|
| **Bennett's modified agar** | | | | |
| Aerial | none | ----- | abundant | (R) 5cb gray yellow pink |
| Vegetative | poor | pale yellow brown<br>no soluble pigment | abundant | [11D4] grayish yellow<br>light brown soluble pigment |
| **Calcium malate agar** | | | | |
| Aerial | none | ----- | poor | (W) a white |
| Vegetative | fair | [7L12] mod. red brown<br>light brown soluble pigment | poor | pale yellow-green<br>pale yellow-green soluble pigment |
| **Czapek's solution agar** | | | | |
| Aerial | poor | (W)a white | none | ----- |
| Vegetative | poor | off white<br>no soluble pigment | none | ----- |
| **Emerson's agar** | | | | |
| Aerial | poor | ----- | abundant | (R)5cb gy. yellow pink |
| Vegetative | abundant | [13L6]<br>no soluble pigment | abundant | [11I5] gy. yellow<br>light brown soluble pigment |

Cultural Characteristics (cont)

| | A21978.6 Growth | A21978.6 Color | S. roseosporus Growth | S. roseosporus Color |
|---|---|---|---|---|
| **Glucose - asparagine agar** | | | | |
| Aerial | good | (W)b white | fair | (W)b white |
| Vegetative | good | [12B2] gy. yellow<br>no soluble pigment | good | [12B2] pale yellow green<br>no soluble pigment |
| **Glycerol - glycine agar** | | | | |
| Aerial | poor | ----- | abundant | (W)b white |
| Vegetative | abundant | [8L12] dark gy. brown<br>brown soluble pigment | abundant | [10G3] light yellow<br>light brown soluble pigment |
| **Nutrient agar** | | | | |
| Aerial | none | ----- | fair | (W)b white |
| Vegetative | poor | pale yellow-gray<br>no soluble pigment | good | pale yellow gray<br>no soluble pigment |
| **Tomato-paste Oatmeal agar** | | | | |
| Aerial | abundant | (R)5cb gy. yell. pink | abundant | (R) 5cb gy. yell. pink |
| Vegetative | abundant | [8L12] dk. gy. brown<br>brown soluble pigment | abundant | [12L7] yellow brown<br>brown soluble pigment |

| Substrate | Carbon Utilization A21978.6 | *S. roseosporus* |
|---|---|---|
| L-Arabinose | + | + |
| D-Fructose | + | - |
| D-Galactose | + | + |
| D-Glucose | + | + |
| i-Inositol | - | - |
| D-Mannitol | + | - |
| D-Raffinose | - | - |
| L-Rhamnose | + | + |
| Salicin | + | + |
| Sucrose | - | - |
| D-Xylose | + | + |

Key:

+ = Positive utilization

- = Negative utilization

| Characteristic | A21978.6 | *S. roseosporus* |
|---|---|---|
| Melanoid Pigments | | |
| ISP #1 (tryptone-yeast ext.) | - | - |
| ISP #6 (peptone-yeast ext. iron) | - | - |
| ISP #7 (tyrosine agar) | - | - |
| ISP #7 mod. (ISP #7 minus tyrosine) | - | - |
| Tyrosine assay | - | - |
| Gelatin liquefaction | + | + |
| Skim milk action | slight hydrolysis | slight hydrolysis |
| Starch hydrolysis | + | + |
| pH range | 5 - 11 | 5 - 11 |
| Temperature range | 25 - 40°C | 25 - 45°C |
| Nitrate reduction | - | + |
| NaCl tolerance; growth up to | 10% | 6% |

**Antibiotic Sensitivity**

| Antibiotic | Conc./Disc | Class | A21978.6 | *S. roseosporus* |
|---|---|---|---|---|
| Erythromycin | 15 µg | Macrolide | + | + |
| Cephalothin | 30 µg | β-Lactam | + | + |
| Lincomycin | 2 µg | Glycoside | - | - |
| Nystatin | 100 units | Polyene | - | - |
| Polymyxin B | 300 units | Peptide | + | - |
| Streptomycin | 10 µg | Aminoglycoside | + | + |
| Tetracycline | 30 µg | Tetracycline | + | + |
| Vancomycin | 30 µg | Glycopeptide | + | + |

+ = sensitive (zones of inhibition)

- = resistant (no zones of inhibition)

Certain characteristics of the A21978.6 strain differ from the characteristics published for S. roseosporus. Culture A21978.6 differs from the published strain in spore size, carrot- and potato-plug growth, NaCl tolerance, and in nitrate reduction.

The A-21978.65 strain of this invention has the identifying characteristics of the A-21978.6 strain, but differs from it in the amount of A-21978C antibiotics produced. The earlier strain, at best, produced no more than 100 mcg of A-21978C antibiotics per mL of fermentation medium. The improved A-21978.65 strain of this invention produces at least 2½ times this amount in tank fermentations and has produced as much as

18 times this amount in shake flask fermentation. With this enhanced A-21978C-producing characteristic, the new strain offers a greatly improved method for obtaining these antibiotics.

As is the case with other organisms, the characteristics of the new A-21978C-producing culture of this invention, Streptomyces roseosporus NRRL 15998, are subject to variation. Recombinants, variants and mutants of the NRRL 15998 strain may be obtained by treatment with various known mutagens such as ultraviolet rays, X-rays, high-frequency waves, radioactive rays and chemicals. Natural and induced variants, mutants and recombinants of Streptomyces roseosporus NRRL 15998 which retain the characteristic of producing the A-21978C antibiotics in high yield also may be used in this invention.

The culture medium used to grow the Streptomyces roseosporus NRRL 15998 culture can be any one of a number of media. For economy in production, optimal yield, and ease of product isolation, however, certain culture media are preferred. Thus, for example, a preferred carbon source in large-scale fermentation is tapioca dextrin, although glucose, fructose, galactose, maltose, mannose, cottonseed oil, methyl oleate, glycerol, refined soybean oil, and the like can also be used. A preferred nitrogen source is enzyme-hydrolyzed casein, although soluble-meat peptone, soybean flour, soybean hydrolysate, soybean grits, yeast, amino acids such as L-asparagine and DL-leucine, and the like are also useful. Nutrient inorganic salts which can be incorporated in the culture media are the soluble salts capable of yielding potassium, ammonium, chloride, sulfate, nitrate and like ions. Among these, $K_2SO_4$ is especially useful for antibiotic production. Molasses ash, ash dialysate and synthetic mineral mix are also useful.

For production of the A-21978C antibiotics, it is preferable to use distilled or deionized water in the fermentation medium. Some of the minerals in tap water, such as, for example, calcium and carbonate, appear to discourage antibiotic production.

Essential trace elements also necessary for the growth and development of the organism should be included in the culture medium. Such trace elements commonly occur as impurities in other constituents of the medium in amounts sufficient to meet the growth requirements of the organism.

Addition of small amounts (e.g., 0.2 ml/L.) of an antifoam agent, such as polypropylene glycol, to large-scale fermentation media may be necessary if foaming becomes a problem.

For production of substantial quantities of the A-21978C antibiotics, submerged aerobic fermentation in tanks is preferred. Small quantities, however, of the A-21978C antibiotics may be obtained by shake-flask culture. Because of the time lag in antibiotic production commonly associated with inoculation of large tanks with the spore form of the organism, it is preferable to use a vegetative inoculum. The vegetative inoculum is prepared by inoculating a small volume of culture medium with the spore form or mycelial fragments of the organism to obtain a fresh, actively growing culture of the organism. The vegetative inoculum is then transferred to a larger tank.

The new A-21978C-producing organism can be grown at temperatures between about 20° and about 40°C. Optimum A-21978C production appears to occur at temperatures of about 30°-32°C.

As is customary in aerobic submerged culture processes, sterile air is dispersed through the culture medium. For efficient production of the A-21978C antibiotics, the percent of air saturation for tank production should be above 20%, preferably above 30% (at 30°C and one atmosphere of pressure).

For tank fermentation, it is preferable to maintain the pH level of the fermentation medium in a range of from about 6.5-7.0. This can be done by the addition of appropriate amounts of, for example, sodium hydroxide (in the early stages) and hydrochloric acid (in the later stages).

Production of the A-21978C antibiotics can be followed during the fermentation by testing samples of the broth or of extracts of the mycelial solids for antibiotic activity against organisms known to be sensitive to the antibiotics. One assay organism useful in testing these antibiotics is Micrococcus luteus. The bioassay is preferably performed by paper-disc assay on agar plates.

Alternatively, the culture solids, including medium constituents and mycelium can be used without extraction or separation, but preferably after removal of water, as a source of the A-21978C antibiotics. For example, after production of A-21978C antibiotic activity, the culture medium can be dried by lyophilization and mixed directly into feed premix.

The formula I compounds are excellent anti-bacterial agents.

In order to illustrate more fully the operation of this invention, the following non-limiting examples are provided.

## Example 1

### Production of the A-21978C Complex

A stock culture is prepared and maintained in the vapor phase of liquid nitrogen. Streptomyces

roseosporus NRRL 15998, previously stored in the vapor phase of liquid nitrogen, was used to inoculate 50 ml of vegetative medium of the following composition:

| Ingredient | Amount (%) |
|---|---|
| Trypticase Soy Broth* | 3.0 |
| Dextrin | 2.5 |
| Water (deionized) | 94.5 |

*Baltimore Biological Laboratories, Cockeysville MD.

The inoculated medium was incubated in a 250-ml Erlenmeyer flask at 30° C for 48 hours on a shaker rotating through an arc of two inches at 250 RPM. The mature vegetative culture was dispensed into multiple containers (0.5 ml/container) and stored in the vapor phase of liquid nitrogen.

In order to provide a larger uniform supply of stored material, one ml of the culture stored in liquid nitrogen was used to inoculate 80 ml of the vegetative medium described above. The inoculated vegetative medium was incubated in a 250-ml Erlenmeyer flask at 30° C for 48 hours on a shaker rotating through an arc two inches in diameter at 250 RPM.

Ten ml of such a culture was used to inoculate 450 ml of a second-stage vegetative growth medium having the same composition as the primary vegetative medium described supra. The second-stage medium was incubated in a 2-liter Erlenmeyer flask for 24 hours at 30° C on a shaker rotating through an arc of 2 inches at 250 RPM.

One liter of the second-stage vegetative culture was used to inoculate 39 liters of sterile tertiary inoculum development medium having the following composition:

| Ingredient | Amount (%) |
|---|---|
| Soybean Flour | 0.5 |
| Yeast Extract[a] | 0.5 |
| Calcium Gluconate | 1.0 |
| KCl[b] | 0.02 |
| $MgSO_4 \cdot 7H_2O$[b] | 0.02 |
| $FeSO_4 \cdot 7H_2O$[b] | 0.0004 |
| Sag 471 (antifoam)[c] | 0.03 |
| Water | 97.9296 |

[a]Difco Laboratories, Detroit MI

[b]Trace minerals were prepared as follows: $FeSO_4 \cdot 7H_2O$ (7.6 g) was dissolved in conc. HCl (76 ml). $MgSO_4 \cdot 7H_2O$ (380 g), KCl (380 g) and deionized water were added to bring the total volume to 3800 ml. To provide the specified minerals, use 80 ml of solution per 39 liters of tertiary inoculum development stage.

[c]Union Carbide, Danbury CT.

The inoculated medium was incubated 24 hours in a stainless steel vessel at 30° C. The vessel was

aerated with sterile air at 0.85 v/v/m and stirred with conventional agitators at 350-450 RPM. The pressure on the vessel was maintained at 5 PSIG.

One liter of the incubated tertiary inoculum stage was used to inoculate 119 liters of sterile production medium having the following composition:

| Ingredient | Amount (%) |
|---|---|
| Soybean Flour | 2.2 |
| $Fe(NH_4)_2SO_4 \cdot 6H_2O$ | 0.066 |
| Dextrose | 0.825 |
| Sag 471 | 0.022 |
| Potato Dextrin | 3.3 |
| Molasses(blackstrap) | 0.275 |
| Tap Water | 93.312 |

The pH was adjusted to 7.0 after addition of the first two ingredients and again after addition of all the ingredients immediately prior to sterilization.

The inoculated production medium was incubated 6 days in a stainless steel vessel at 30 °C and aerated with sterile air at a rate of 0.5 v/v/m. The medium was stirred with conventional agitators at 250 RPM from 0 to 15 hours and at 350 RPM after 15 hours. The pH was maintained at or above 6.5 by addition of ammonium hydroxide solution. The yield of A-21978C complex was 0.282 grams per liter of broth at the end of the fermentation. The factor distribution is described in Table 1.

Example 2

Enhanced Production of A-21978$C_8$

The primary, secondary, and the tertiary growth stages were carried out as described in Example 1. The production stage was initiated as described in Example 1 except, beginning at 28 hours a sterile solution consisting of 50% v/v caprylic (octanoic) acid and methyl oleate was fed to the fermentation at a rate of 0.13 ml per liter of fermentation broth per hour and maintained at this rate until termination of the fermentation at 144 hours. The yield of A-21978C complex was 1.255 grams per liter of broth, a 445% increase in yield over that in Example 1. Factor A-21978$C_8$ (the formula I compound wherein R = octanoyl) represented 9% of the total A-21978C complex prepared by this method; no A-21978$C_8$ was detected in A-21978C complex prepared by the method of Example 1.

Example 3

Enhanced Production of A-21978$C_9$

The primary, secondary and tertiary inoculum development stages were carried out as described in Example 1. The production stage was initiated as described in Example 1 except, beginning at 28 hours, a sterile solution consisting of 25% v/v nonanoic acid, and 75% methyl oleate was fed to the fermentation at a rate of 0.13 ml per liter of fermentation broth per hour and maintained at this rate until termination of the fermentation at 144 fours. The yield of A-21978C complex was 0.821 grams per liter of broth, a 293% increase over that obtained in Example 1. Factor A-21978$C_9$ (formula I: R = nonanoyl) represented 10% of the total A-21978C complex prepared by this method; no A-21978$C_9$ was detected in the A-21978C complex prepared by the method of Example 1.

Example 4

Enhanced Production of A-21978$C_{10}$ Factor (formula I: R = n-decanoyl)

The primary and second stage vegetative growth stages were cultured as described in Example 1. In the tertiary stage 800 ml of secondary inoculum culture were used to inoculate 950 liters of sterile tertiary inoculum development medium having the following composition:

| Ingredient | Amount (%) |
|---|---|
| Dextrose | 2.0 |
| Calcium Carbonate | 0.2 |
| Soybean Flour | 2.0 |
| Yeast Extract | 0.1 |
| KCl[a] | 0.02 |
| $MgSO_4 \cdot 7H_2O$[a] | 0.02 |
| $FeSO_4 \cdot 7H_2O$[a] | 0.0004 |
| Sag 471 (antifoam) | 0.02 |
| Water | 95.6396 |

[a]Trace mineral solution prepared as described in Example 1.

The inoculated medium was incubated 24 hours in a stainless steel vessel at 30°C. The vessel was aerated with sterile air at a rate of 0.8 v/v/m and stirred with conventional agitators. One liter of this tertiary stage inoculum was used to inoculate 119 liters of production stage medium having the composition described in Example 1. The production stage was also initiated as described in Example 1 except, beginning at 28 hours, a sterile solution consisting of 50% v/v capric (decanoic) acid and 50% methyl oleate was fed to the fermentation at a rate of 0.26 ml per liter of fermentation broth per hour and maintained at this rate until termination of the fermentation at 283 hours. The yield of A-21978C complex was 1.94 grams per liter of broth, a 687% increase over that obtained in Example 1. The concentration of the A-21978$C_{10}$ factor (formula I: R = n-decanoyl) was 1.63 grams Per liter or 84% of the total A-21978C complex. This was 13583% greater than the amount of A-21978$C_{10}$ produced using the Example 1 procedure.

Example 5

Alternate Method of Enhanced Production of A-21978$C_{10}$

The primary, secondary, and tertiary inoculum development stages were carried out as described in Example 1. The production stage was initiated as described in Example 1 except, beginning at 28 hours, a sterile solution consisting of 25% v/v capric acid ethyl ester (ethyl caprate) and 75% methyl oleate was fed to the fermentation at a rate of 0.13 ml per liter of fermentation broth per hour and maintained at this rate until termination of the fermentation at 144 hours. The yield of A-21978C complex was 1.022 grams per liter, a 362% increase over that obtained in Example 1. The concentration of factor A-21978$C_{10}$ was 0.202 grams per liter or 20% of the total A-21978C complex. This was 1683% greater than the concentration of A-21978$C_{10}$ produced using the method of Example 1.

Example 6

Alternate Method for Enhanced Production of A-21978$C_{10}$

The primary and second stage vegetative growth stages were cultured as described in Example 1. The tertiary stage inoculum was cultured as described in Example 4 except that the volume of medium was 1900 liters and the duration of the stage was extended to 48 hours. The aeration rate was 0.3 v/v/m from 0 to 24 hours, 0.45 v/v/m from 24 to 40 hours and 0.90 v/v/m from 40 to 48 hours. The production stage was initiated as described in Example 1. At 23 hours a sterile slurry of 0.004% yeast extract was batch fed to the fermentation. Beginning at 36 hours, a solution of glycerol and ammonium decanoate was fed at a rate of 0.84 ml per liter of fermentation broth per hour. The feeding solution contained glycerol (3600 g), deionized water (9000 ml), capric acid (1 liter), and concentrated ammonium hydroxide solution (620 ml). The feed was maintained at this rate until the fermentation was terminated at 143 hours. The yield of A-21978C complex was 1.772 grams per liter, a 628% increase over that obtained in Example 1. The concentration of factor A-21978$C_{10}$ was determined to be 0.739 grams per liter or 42% of the total A-21978C complex. This was 6158% greater than the concentration of A-21978$C_{10}$ when produced by the method of Example 1.

Example 7

Enhanced Production of A-21978$C_{11}$

The primary, secondary, and tertiary inoculum stages were carried out as described in Example 1. The production stage was initiated as described in Example 1 except, beginning at 28 hours, a sterile solution consisting of 25% v/v undecanoic acid and 75% methyl oleate was fed to the fermentation at a rate of 0.13 ml per liter of fermentation broth per hour until termination of the fermentation at 144 hours. The yield of A-21978C complex was 1.62 grams per liter, a 574% increase over that obtained in Example 1. The concentration of factor A-21978$C_{11}$ (formula I: R = undecanoyl) was determined to be 0.70 grams per liter or 43% of the total A-21978C complex. Factor A-21978$C_{11}$ could not be detected in the A-21978C complex prepared by the Example 1 method.

Example 8

Enhanced Production of A-21978$C_5$

The primary, secondary, and tertiary inoculum stages were carried out as described in Example 1. The production stage was initiated as described in Example 1 except, beginning at 28 hours, a sterile solution consisting of 25% v/v lauric acid and 75% methyl oleate was fed to the fermentation at a rate of 0.13 ml per liter of fermentation broth per hour until termination of the fermentation at 144 hours. The yield of A21978C complex was 1.12 grams per liter, a 400% increase over that obtained in Example 1. The concentration of factor A-21978$C_5$ (formula I: R = dodecanoyl) was determined to be 0.372 grams per liter or 33% of the total A-21978C complex. This was 5314% greater than the concentration of A-21978$C_5$ found in A-21978C complex produced by the method of Example 1.

Table 1.

EFFECT OF VARIOUS LIPID SUBSTRATES ON THE PRODUCTION of A-21978C COMPONENT

| Example | Lipid Substrate | A-21978C Component (μg/ml)[a,b] $C_1$ | $C_2$ | $C_3$ | $C_5$ | $C_8$ | $C_9$ | $C_{10}$ | $C_{11}$ |
|---|---|---|---|---|---|---|---|---|---|
| 1 | None | 77 | 113 | 72 | 7 | -- | -- | 12[c] | -- |
| 2 | Caprylic Acid | 276 | 312 | 214 | 175 | 113 | -- | 170 | -- |
| 3 | Nonanoic Acid | 147 | 177 | 125 | 192 | -- | 83 | 90 | -- |
| 4 | Capric Acid | 135 | 50 | 48 | 77 | -- | -- | 1630[d] | -- |
| 5 | Capric Acid Ethyl Ester | 168 | 246 | 156 | 251 | -- | -- | 202[d] | -- |
| 6 | Ammonium Decanoate | 335 | 371 | 228 | 98 | -- | -- | 739[d] | -- |
| 7 | Undecanoic Acid | 163 | 206 | 158 | 273 | -- | -- | 118 | 700 |
| 8 | Lauric Acid | 204 | 236 | 141 | 372 | -- | -- | 173 | -- |

[a] The concentration of the antibiotic components in filtered broth was estimated by high performance liquid chromatography; the various components were detected by ultraviolet light absorption.

[b] $C_0$, $C_1$, $C_2$, $C_3$ and $C_5$ = naturally occurring A-21978C factors; $C_8$, $C_9$, $C_{10}$ and $C_{11}$ = formula I compounds wherein R = $C_8$, $C_9$, $C_{10}$ and $C_{11}$ acyl groups, respectively.

[c] Natural factor $C_0$

[d] found to be substantially R = n-decanoyl

## Example 9

### Alternate Production of the A-21978C Complex

A-21978C complex is produced using the procedure of Example 1, but the *Streptomyces roseosporus* NRRL 11379 culture is used.

## Example 10

Alternate Method for Enhanced A-21978$C_{10}$ Production

A-21978$C_{10}$ is produced using the method of Example 6, but the Streptomyces roseosporus NRRL 11379 culture is used.

Example 11

Shake-Flask Production of the A-21978C Complex

The general procedure described in Example 1 is used, but under shake flask conditions with the following fermentation medium:

| Ingredient | Amount (g/L) |
|---|---|
| Glucose | 7.5 |
| Dextrin[a] | 30 |
| Enzyme-hydrolyzed casein[b] | 5 |
| Peptone[c] | 5 |
| Molasses | 2.5 |
| Deionized water | q.s. to 1 L |

[a]Stadex 11, A.E. Staley Co., Decatur IL

[b]NZ Amine A, Humko Sheffield Chemical, Lyndhurst NJ

[c]Biosate, Baltimore Biological Laboratories, Cockeysville MD

The Yield of A-21978C complex from this fermentation was 1800 mcg per mL of broth.

**Claims**

**1.** A process for preparing an A-21978C derivative of the formula (I):

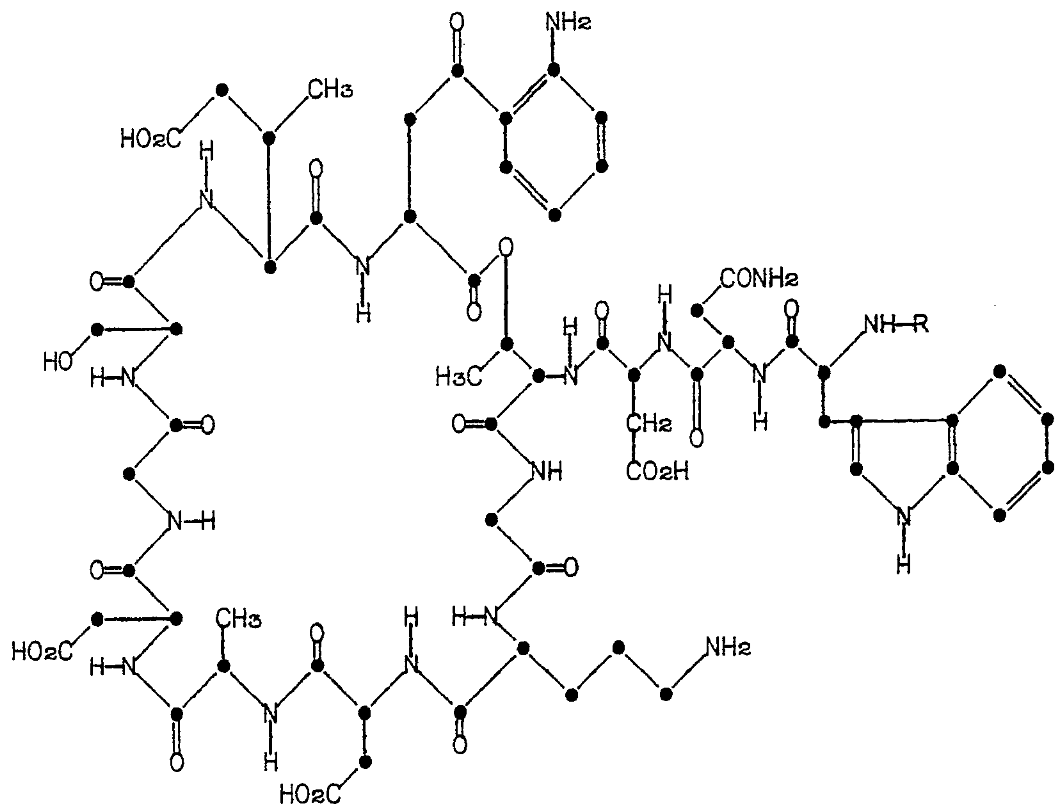

(I)

in which R is a $C_2$-$C_{14}$-alkanoyl group, which comprises feeding the corresponding $C_2$-$C_{14}$ alkanoic acid, ester or salt thereof, to an A-21978C-producing culture.

2. A process as claimed in claim 1 in which a $C_2$-$C_{14}$-alkanoic acid is used.

3. A process as claimed in claim 1 in which a $C_1$-$C_4$-alkyl ester of the $C_2$-$C_{14}$-alkanoic acid is used.

4. A process as claimed in claim 1 in which a salt of the $C_2$-$C_{14}$-alkanoic acid is used.

5. A process as claimed in any one of claims 1 to 4 in which capric acid, an ester or salt thereof is used.

6. A process as claimed in claim 1 in which the $C_2$-$C_{14}$ alkanoic acid used is caprylic acid, nonanoic acid, undecanoic acid, lauric acid or an ester or salt thereof.

7. A process as claimed in any one of claims 1 to 6 in which the A-21978C-producing culture comprises Streptomyces roseosporus NRRL 11379, or a mutant, variant, or recombinant thereof retaining the characteristic of producing said antibiotic in high yield.

8. A process as claimed in claim 7 in which the culture comprises Streptomyces roseosporus NRRL 15998, or an A-21978C producing mutant, variant or recombinant thereof.

9. A process for preparing the antibiotic A-21978C complex or factors which comprises cultivating Streptomyces roseosporus NRRL 15998, or an A-21978C-producing mutant, variant or recombinant thereof retaining the characteristic of producing said antibiotic in high yield, in a culture medium containing assimilable sources of carbon, nitrogen, and inorganic salts, under submerged aerobic

fermentation conditions until the A-21978C antibiotics are produced, and if desired, separating the A-21978C complex produced, and further, if desired, separating the individual A-21978C factors from the complex.

10. A process for preparing the antibiotic A-21978C complex as claimed in claim 9 which includes the additional step of separating the A-21978C complex from the culture medium.

11. A process for preparing the individual antibiotic A-21978C factors $C_0$, $C_1$, $C_2$, $C_3$, $C_4$ or $C_5$ as olaimed in claim 9 or 10 which includes the additional step of separating the individual factors from the A-21978C antibiotic complex.

12. A biologically pure culture of Streptomyces roseosporus NRRL 15998, or a mutant, variant or recombinant thereof retaining the characteristic of producing the A-21978C antibiotics in high yield.

13. Streptomyces roseosporus NRRL 15998.

**Revendications**

1. Procedé pour la préparation d'un dérivé de A-21978C répondant à la formule (I) :

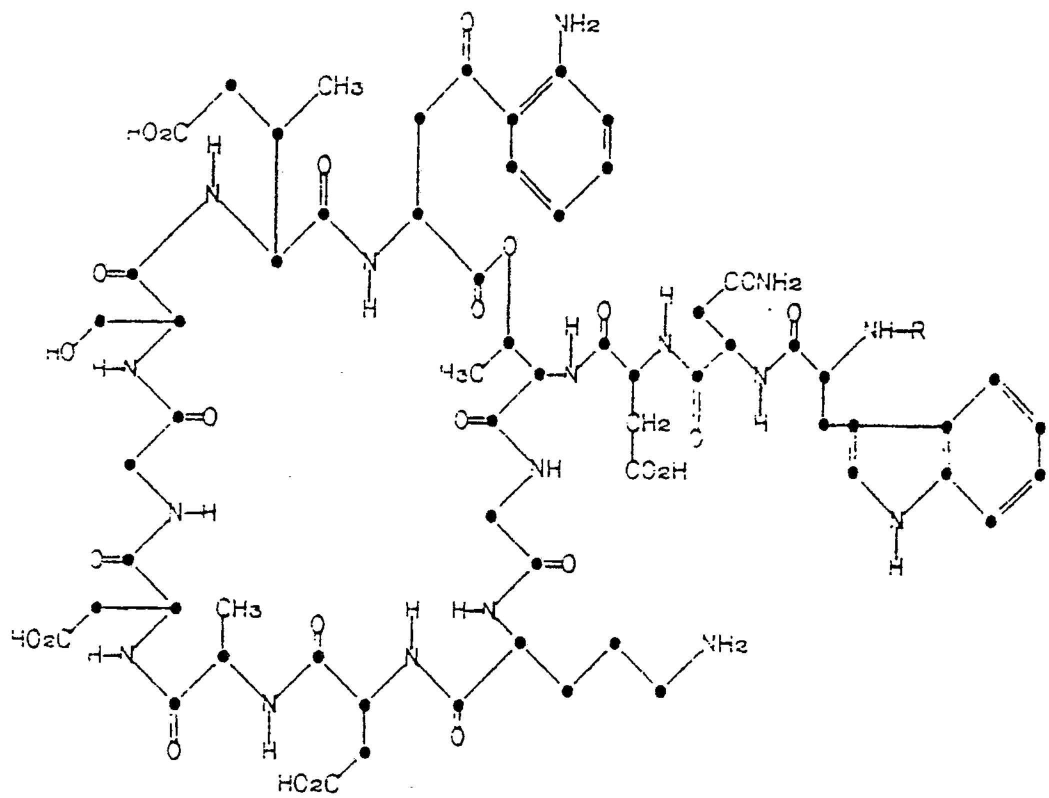

dans laquelle R représente un groupe alcanoyle en $C_2$-$C_{14}$, ce procédé consistant à procurer 1'acide alcanoïque en $C_2$-$C_{14}$ correspondant, son ester ou son sel, à une culture productrice de A-21978C.

2. Procédé selon la revendication 1, dans lequel on utilise un acide alcanoïque en $C_2$-$C_{14}$.

3. Procédé selon la revendication 1, dans lequel on utilise un ester alkylique en $C_1$-$C_4$ de l'acide alcanoïque en $C_2$-$C_{14}$.

4. Procédé selon la revendication 1, dans lequel on utilise un sel de l'acide alcanoïque en $C_2$-$C_{14}$.

**5.** Procédé selon l'une quelconque des revendications 1 à 4, dans lequel on utilise l'acide caprique, un de ses esters ou un de ses sels.

**6.** Procédé selon la revendication 1, dans lequel l'acide alcanoïque en $C_2$-$C_{14}$ utilisé est l'acide caprylique, l'acide nonanoïque, l'acide undécanoïque, 1'acide laurique ou un ester ou un sel de ces derniers.

**7.** Procédé selon l'une quelconque des revendications 1 à 6, dans lequel la culture productrice de A-21978C comprend **Streptomyces roseosporus** NRRL 11379 ou un mutant, un variant ou encore un recombinant de ce dernier, qui garde la caractéristique de produire l'antibiotique avec un rendement élevé.

**8.** Procédé selon la revendication 7, dans lequel la culture comprend **Streptomyces roseosporus** NRRL **15998** ou un mutant, un variant ou encore un recombinant de ce dernier, producteur de A-21978C.

**9.** Procédé pour la préparation du complexe antibiotique A-21978C ou de ses facteurs, consistant à cultiver **Streptomyces roseosporus** NRRL **15998** ou un mutant, un variant ou encore un recombinant de ce dernier, producteur de A-21978C, qui garde la caractéristique de produire l'antibiotique avec un rendement élevé, dans un milieu de culture contenant des sources assimilables de carbone, de l'azote, ainsi que des sels inorganiques, dans des conditions de fermentation aérobie submergée jusqu'à 1'obtention des antibiotiques A-21978C et si on le souhaite, séparer le complexe A-21978C obtenu et encore, si on le souhaite, séparer les facteurs individuels de A-21978C du complexe.

**10.** Procédé pour la préparation du complexe antibiotique A-21978C selon la revendication 9, qui implique l'étape supplémentaire consistant à séparer le complexe A-21978C du milieu de culture.

**11.** Procédé pour la préparation des facteurs individuels de l'antibiotique A-21978C $C_0$, $C_1$, $C_2$, $C_3$, $C_4$ ou $C_5$ selon la revendication 9 ou 10, qui englobe l'étape supplémentaire de séparer les facteurs individuels du complexe antibiotique A-21978C.

**12.** Culture biologiquement pure de **Streptomyces roseosporus** NRRL **15998** ou d'un de ses mutants, variants ou recombinants, qui garde la caractéristique de produire les antibiotiques A-21978C avec un rendement élevé.

**13.** **Streptomyces roseosporus** NRRL **15998.**

## Patentansprüche

**1.** Verfahren zur Herstellung eines A-21978C-Derivats der Formel (I):

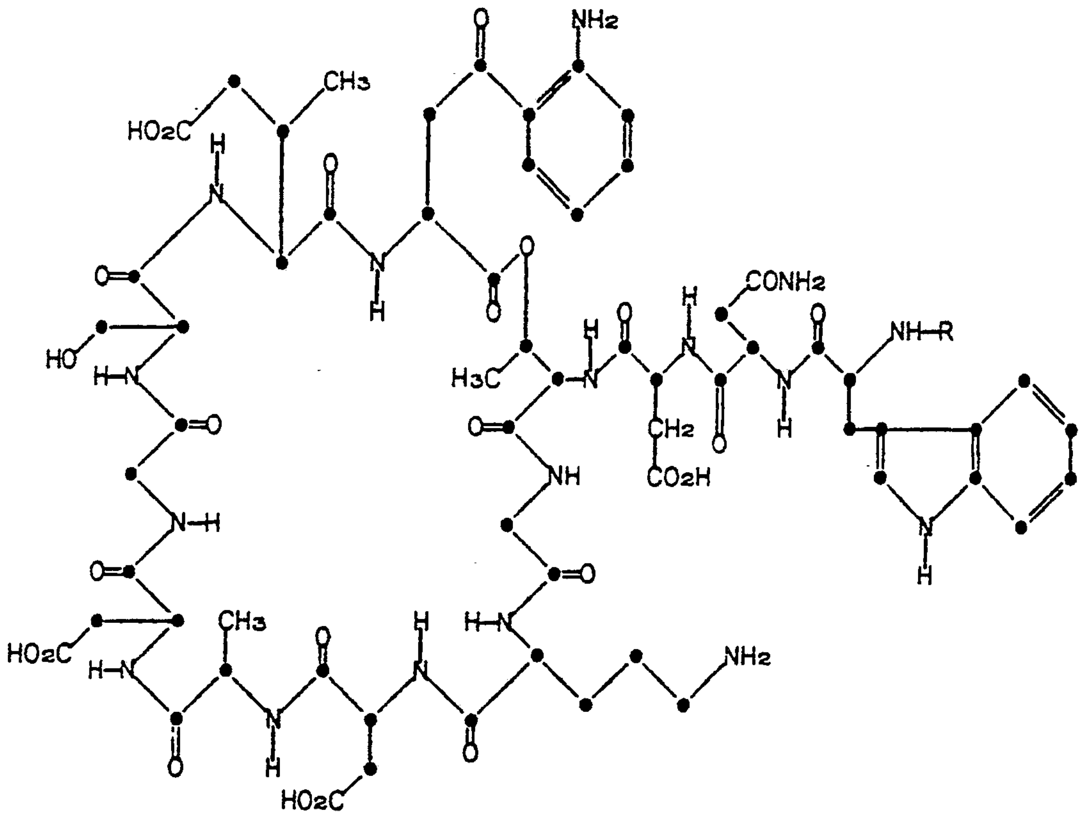

(I)

worin R eine $C_2$-$C_{14}$-Alkanoylgruppe ist, umfassend, daß man die entsprechende $C_2$-$C_{14}$-Alkansäure, einen Ester oder ein Salz davon einer A-21978C-produzierenden Kultur zuführt.

2. Verfahren nach Anspruch 1, worin eine $C_2$-$C_{14}$-Alkansäure verwendet wird.

3. Verfahren nach Anspruch 1, worin ein $C_1$-$C_4$-Alkylester der $c_2$-$C_{14}$-Alkansäure verwendet wird.

4. Verfahren nach Anspruch 1, worin ein Salz der $C_2$-$C_{14}$-Alkansäure verwendet wird.

5. Verfahren nach einem der Ansprüche 1 bis 4, worin Caprinsäure, ein Ester oder ein Salz davon verwendet wird.

6. Verfahren nach Anspruch 1, worin die verwendete $C_2$-$C_{14}$-Alkansäure Caprylsäure, Nonansäure, Undecansäure, Laurinsäure oder ein Ester oder Salz davon ist.

7. Verfahren nach einem der Ansprüche 1 bis 6, worin die A-21978C-produzierende Kultur Streptomyces roseosporus NRRL 11379 oder eine Mutante, Variante oder Rekombinante davon, die die Eigenschaft der Herstellung dieses Antibiotikums in hoher Ausbeute aufweist, umfaßt.

8. Verfahren nach Anspruch 7, worin die Kultur Streptomyces roseosporus NRRL 15998 oder eine A-21978C-produzierende Mutante, Variante oder Rekombinante davon umfaßt.

9. Verfahren zur Herstellung des antibiotischen A-21978C-Komplexes oder dessen Faktoren, umfassend, daß man Streptomyces roseosporus NRRL 15998 oder eine A-21978C-produzierende Mutante, Variante

oder Rekombinante davon, die die Eigenschaft der Herstellung dieses Antibiotikums in hoher Ausbeute aufweist, in einem Kulturmedium, das assimilierbare Quellen für Kohlenstoff, Stickstoff und anorganische Salze enthält, unter aeroben Submersfermentationsbedingungen züchtet, bis A-21978C-Antibiotika produziert werden und, falls erwünscht, den hergestellten A-21978C-Komplex abtrennt und weiterhin, falls erwünscht, die einzelnen A-21978C-Faktoren von dem Komplex trennt.

**10.** Verfahren zur Herstellung des antibiotischen A-21978C-Komplexes nach Anspruch 9, das die zusätzliche Stufe einschließt, daß man den A-21978C-Komplex von dem Kulturmedium abtrennt.

**11.** Verfahren zur Herstellung der einzelnen antibiotischen A-21978C-Faktoren $C_0$, $C_1$, $C_2$, $C_3$, $C_4$ oder $C_5$ wie in Anspruch 9 oder 10 beansprucht, das die zusätzliche Stufe einschließt, daß man die einzelnen Faktoren von dem A-21978C-antibiotischen Komplex abtrennt.

**12.** Biologisch reine Kultur von Streptomyces roseosporus NRRL 15998 oder eine Mutante, Variante oder Rekombinante davon, die die Eigenschaft der Produktion von A-21978C-Antibiotika in hoher Ausbeute aufweist.

**13.** Streptomyces roseosporus NRRL 15998.